# EUROPÄISCHE PATENTANMELDUNG

(11) **EP 0 888 768 A2**
(43) Veröffentlichungstag der Anmeldung: **07.01.1999**
(21) Anmeldenummer: 98106188.0
(22) Anmeldetag: 04.04.1998
(51) Int. Cl.: A61K 7/13, D06P 3/14

(54) **Haarfärbemittel**

(30) Priorität: 03.07.1997 DE 19728389
(71) Anmelder: Wella Aktiengesellschaft, 64274 Darmstadt (DE)
(72) Erfinder: Braun, Hans-Jürgen, Dr., 3182 Überstorf (CH); Semadeni, Pascal André, Dr., 1792 Cordast (CH)

(57) **Zusammenfassung**

Die vorliegende Erfindung betrifft ein Mittel zur Färbung keratinischer Fasern, enthaltend mindestens ein Nitrobenzylderivat der Formel (I) 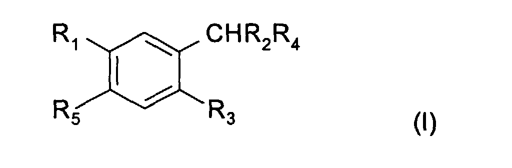 worin unabhängig voneinander gilt:
- R₁ =: H, NH₂, NHRₓ, NRₓ, NO₂, CH(CN)₂, CONHRₓ,
OH, ORₓ, OCORₓ, CHRₓCOORₓ, CHCNCOORₓ oder CORₓ
mit Rₓ = H, Phenyl oder einer niederen Alkylgruppe mit
1-4 C Atomen;
- R₂ =: CN, CONHRₓ, NHCORₓ, CORₓ, COORₓ, Trihydroxypyrimidinyl oder 4,6-Dihydroxy-2-mercaptopyrimidinyl
mit Rₓ = H, Phenyl oder einer niederen Alkylgruppe mit
1-4 C Atomen;
- R₄ =: CN, CONHRₓ, NHCORₓ, CORₓ oder COORₓ
mit Rₓ = H, Phenyl oder einer niederen Alkylgruppe mit
1-4 C Atomen; und
- R₃ =: H oder NO₂,
- R₅ =: H oder NO₂ , wobei R₃ und R₅ nicht gleichzeitig H bedeuten;
sowie ein Verfahren zum Färben von keratinischen Fasern unter Verwendung dieses Mittels.

## Beschreibung

Gegenstand der Erfindung sind Mittel zur Färbung von Fasern, insbesondere keratinischer Fasern, mit Nitrofarbstoffen, wobei als Nitrofarbstoffe Nitrobenzylderivate verwendet werden. Als keratinhaltige Fasern kommen zum Beispiel Wolle, Pelze und menschliche Haare in Betracht.

Für die Haarfärbung haben Nitrofarbstoffe eine wesentliche Bedeutung erlangt. Durch Kombination verschiedener Nitrofarbstoffe lassen sich Färbemittel herstellen, die ohne Zusatz von Oxidationsmitteln Haare in natürlichen und modischen Tönen zu färben vermögen. Es besteht daher in jedem Fall ein Bedarf nach gelben bis orangen Nitrofarbstoffen. An diese Farbstoffe werden viele Anforderungen gestellt. So müssen sie in toxikologischer und dermatologischer Hinsicht unbedenklich sein und die Erzielung von Färbungen in der gewünschten Intensität ermöglichen, was unter anderem auch eine ausreichende Wasserlöslichkeit voraussetzt. Ausserdem wird für die erzielten Haarfärbungen eine gute Lichtechtheit, Dauerwellechtheit und Reibechtheit gefordert. Andererseits dürfen die Farbstoffe bei der Nachbehandlung oder bei der Haarwäsche nicht zu leicht wieder ausgewaschen werden.

Für die Erzielung gelber bis oranger Färbungen, die den oben beschriebenen Anforderungen entsprechen, werden beispielsweise Nitroalkylbenzole eingesetzt. Ihre Verwendung ist jedoch infolge mangelhafter Löslichkeit eingeschränkt.

Nitroaniline beziehungsweise Aminonitrophenole, wie sie beispielsweise in der DE-AS 1 619 395 beziehungsweise im International Journal of Cosmetic Science 1982, Seite 25 - 35 beschrieben werden, erfüllen zwar zum Teil die vorstehend genannten Voraussetzungen, jedoch sind ihre physiologischen Eigenschaften nicht befriedigend.

Es wurde nun überraschend gefunden, daß bestimmte Nitrobenzylderivate Fasern, insbesondere Keratinfasern, wie zum Beispiel Haare, intensiv orange bis rot oder braun färben.

Gegenstand der vorliegenden Erfindung ist deshalb ein Mittel zur Färbung von Fasern, insbesondere keratinischer Fasern, welches dadurch gekennzeichnet ist, daß es mindestens ein Nitrobenzylderivat der Formel (I), 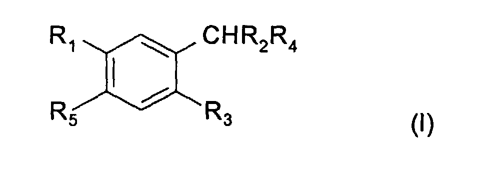 worin unabhängig voneinander gilt:
- R₁ =: H, NH₂, NHRₓ, NRₓ, NO₂, CH(CN)₂, CONHRₓ,
OH, ORₓ, OCORₓ, CHRₓCOORₓ, CHCNCOORₓ oder CORₓ
mit Rₓ = H, Phenyl oder einer niederen Alkylgruppe mit
1-4 C Atomen;
- R₂ =: CN, CONHRₓ, NHCORₓ, CORₓ, COORₓ, Trihydroxypyrimidinyl oder 4,6-Dihydroxy-2-mercaptopyrimidinyl
mit Rₓ = H, Phenyl oder einer niederen Alkylgruppe mit
1-4 C Atomen;
- R₄ =: CN, CONHRₓ, NHCORₓ, CORₓ oder COORₓ
mit Rₓ = H, Phenyl oder einer niederen Alkylgruppe mit
1-4 C Atomen; und
- R₃ =: H oder NO₂,
- R₅ =: H oder NO₂ , wobei R₃ und R₅ nicht gleichzeitig H bedeuten; enthält.

Bevorzugte Nitrobenzylderivate gemäß der allgemeinen Formel (I) sind solche mit R₁ = H, R₂ = CN oder COOEt, R₃ = H oder NO₂, R₄ = CN oder COOEt und R₅ = NO₂, beispielsweise Cyano-(2,4-dinitrophenyl)-essigsäureethylester, (5-Amino-2,4-dinitrophenyl)-cyano-essigsäureethylester, Cyano-[2,4-Dinitro-5-(cyano-ethoxycarbonylmethyl)-phenyl]-essigsäureethylester, 2-(2,4-Dinitrophenyl)-3-oxo-butyrylessigsäureethylester, 2-(2,4-Dinitrophenyl)-malonsäurediethylester, Cyano-4-nitrophenyl-essigsäureethylester, 2-(2,4-Dinitrophenyl)-malonodinitril, 2-(4-Nitrophenyl)-malonsäurediethylester.

Die Verbindungen der Formel (I) werden in dem erfindungsgemäßen Mittel in einer Gesamtmenge von etwa 0,01 bis 10 Gewichtsprozent, insbesondere 0,5 bis 10 Gewichtsprozent, eingesetzt.

Die Verbindungen der Formel (I) färben keratinisches Material ohne den Zusatz weiterer Farbstoffe in intensiv orange-roten Farbtönen, wobei Variationsmöglichkeiten und eine Nuancenvielfalt in allen Abstufungen von hellorange bis rotbraun möglich ist.

Zur Erzielung weiterer Farbnuancen können zusätzlich übliche direktziehende Farbstoffe, beispielsweise Nitrofarbstoffe, Azofarbstoffe, Anthrachinonfarbstoffe oder Triphenylmethanfarbstoffe, alleine oder im Gemisch miteinander, zugesetzt werden.

Beispiele für geeignete Nitrofarbstoffe sind 1,4-Bis[(2'-hydroxyethyl)-amino]-2-nitrobenzol, 1-(2'-Hydroxyethyl)amino-2-nitro-4-bis-(2''-hydroxyethyl)amino-benzol (HC Blue No. 2), 1-Amino-3-methyl-4-(2'-hydroxyethyl)amino-6-nitro-benzol (HC Violet No. 1), 4,N-Ethyl,N-(2''-hydroxyethyl)amino-1-(2''-hydroxyethyl)amino-2-nitro-benzol-hydrochlorid (HC Blue No. 12), 4-Bis-(2'-hydroxyethyl)amino-1-(2''-methoxyethyl)amino-2-nitrobenzol HC Blue No. 11), 1-(2',3'-Dihydroxypropyl)amino-2-nitro-4-[N-methyl-(2''-hydroxyethyl)-amino]-benzol-Hydrochlorid (HC Blue No. 10), 1-[(2',3'-Dihydroxypropyl)amino]-2-nitro-4-[N-ethyl-2''-(hydroxyethyl)-amino]-benzol-Hydrochlorid (HC Blue No. 9), 1-(3'-Hydroxypropylamino)-2-nitro-4-bis-(2''-hydroxyethylamino)-benzol (HC Violet No. 2), 4,N-Methyl,N-(2',3'-dihydroxypropyl)amino-1-methylamino-2-nitro-benzol-Hydrochlorid (HC Blue No. 6), 4'Amino-2'-nitro-2''-carboxy-4''-dimethylamino-diphenylamin (HC Blue No. 13), 1-Amino-4-(2'-hydroxyethyl)-amino-2-nitrobenzol (HC Red No. 7), 4-Amino-2-nitro-diphenylamin (HC Red No. 1), 1-Amino-2-nitro-4-bis-(2'-hydroxyethyl)-amino-benzol-Hydrochlorid (HC Red No. 13), 1-Amino-2-nitro-4-(2'-hydroxyethyl)amino-5-chlorbenzol, 1-(2'-Hydroxyethyl)amino-2-nitro-4-amino-benzol (HC Red No. 3), 1-Hydroxy-3-nitro-4-amino-benzol, 1-Hydroxy-3-nitro-4-(2'-hydroxyethylamino)benzol, 1-(2'-Aminoethyl)amino-2-nitro-4-(2'-hydroxyethoxy)-benzol (HC Orange No. 2), 3-Nitro-4-(2'-hydroxyethyl)amino-phenylglycerinether (HC Orange No. 3), 1-Amino-5-chlor-4-(2',3'-dihydroxypropyl)amino-2-nitro-benzol (HC Red No. 10), 1,4-Bis-[(2',3'-dihydroxypropyl)amino]-5-chlor-2-nitro-benzol (HC Red No. 11), 1-Hydroxy-2-(2'hydroxyethyl)amino-4,6-dinitro-benzol, 3-Nitro-4-ethylamino-benzoesäure, 4-Amino-2-nitro-diphenylamino-2-carbonsäure, 2-Chlor-6-ethylamino-4-nitrophenol, 2-Amino-6-chlor-4-nitrophenol, 1-Hydroxy-3-nitro-4-(3'-hydroxypropylamino)-benzol, 2,5-Diamino-6-nitropyridin, 1,2,3,4-Tetrahydro-6-nitrochinoxalin, 7-Amino-3,4-dihydro-6-nitro-2H-1,4-benzoxazin (HC Red 14), 1-Amino-2-(2'-hydroxyethyl)amino-5-nitro-benzol (HC Yellow No. 5), 1-(2'-Hydroxyethoxy)-2-(2''-hydroxyethyl)amino-5-nitro-benzol (HC Yellow No. 4), 1-(2'-Hydroxyethyl)-amino-2-nitro-benzol (HC Yellow No. 2), 1-Methoxy-2-(2'-hydroxyethyl)-amino-5-nitro-benzol, 1-Hydroxy-2-amino-3-nitro-benzol, 1-Amino-2-methyl-6-nitro-benzol, 1-(2'Hydroxyethyl)-oxy-3-methylamino-4-nitro-benzol, 1-Methylamino-2-nitro-5-(2',3'-dihydroxypropyl)-oxybenzol, 1-(2'-Hydroxyethyl)amino-2-hydroxy-4-nitro-benzol (HC Yellow No. 11), 1-Methoxy-3-(2'-aminoethyl)-amino-4-nitro-benzol-Hydrochlorid (HC Yellow No.9), 1-(2'-Ureidoethyl)amino-4-nitro-benzol, 4-(2',3'-Dihydroxypropyl)-amino-3-nitro-trifluormethyl-benzol (HC Yellow No. 6), 2,4-Bis-[N-(2'-hydroxyethyl)amino]-5-chlor-nitrobenzol (HC Yellow No. 10), 4-(2'-Hydroxyethyl)amino-3-nitro-methylbenzol, 4-(2'-Hydroxyethyl)amino-3-nitro-chlorbenzol (HC Yellow No. 12), 4-(2'-Hydroxyethyl)amino-3-nitro-trifluormethyl-benzol (HC Yellow No. 13), 4-(2'-Hydroxyethyl)amino-3-nitro-benzonitril (HC Yellow No. 14), 4-(2'-Hydroxyethyl)amino-3-nitro-benzamid (HC Yellow No. 15), 4-(Di(2-hydroxyethyl)amino)-2-nitroanilin, N-(2-Hydroxyethyl)-4-methyl-2-nitroanilin, 4-(2',3'-Dihydroxypropyl)-amino-3-nitro-trifluormethylbenzol, 4-(2'-Hydroxyethyl)-amino-3-nitro-chlorbenzol, 1-Hydroxy-2-amino-4,6-dinitro-benzol und 4-(2'-Hydroxyethyl)-amino-3-nitro-methylbenzol, genannt werden.

Beispiele für geeignete Azofarbstoffe sind 1-(4'-Nitrophenylazo)-2-methyl-4-bis-(2'-hydroxyethyl)amino-benzol, 1-(3'-Nitro-4-amino)-phenylazo-2-hydroxy-7-trimethyl-ammoniumchlorid-naphthalin, 1-(2'Hydroxy-4'sulfo-6'nitro)-naphthylazo-2-hydroxynaphthalin CI 15700, 1-(4'-Amino-phenylazo)-2-methyl-4-bis-[(2'-hydroxyethyl)-amino]-benzol, 5-(4'-Dimethylaminophenylazo)-1,4-dimethyl-triazoniumchlorid, 1-(2'-Methoxyphenylazo)-2-hydroxy-7-trimethylammonium-naphthalinchorid, 1-(4'-Aminophenylazo)-2-hydroxy-7-trimethylammonium-naphthalin, 4-(3'-Trimethylammoniumphenylazo)-N-phenyl-3-methyl-pyrazolon (5), 4-Hydroxy-3-[(4'-sulfo-1'-naphthyl)azo]-1-naphthalinsulfonsäure, 1-(4'-Sulfophenylazo)-2-hydroxynaphthalin, 1-(4'-Sulfonphenylazo)-2-hydroxy-6-sulfo-naphthalin CI 15985, 4-Amino-[4'-bis-(2''-hydroxyethyl)amino]-azobenzol, 4-Amino-[4'-bis-(2''-hydroxyethyl)amino]-2'-methyl-azobenzol, 3-(2',6'-Diaminopyridyl-3'-azo)-pyridin, 7-Phenylazo-1-amino-3,6-disulfo-8-hydroxy-naphthalin, 5-Acetylamino-4-hydroxy-3-[(2'-methylphenyl)azo]-2,7-naphthalin-disulfonsäure und 2-(2',4'-Dimethylphenylazo)-6-(4''-sulfophenylazo)-1,3-dihydroxybenzol.

Beispiele für geeignete Anthrachinonfarbstoffe sind 1,4-Bis-(2',3'-dihydroxypropyl)amino-anthrachinon, 1-Methylamino-4-(2'-hydroxyethyl)amino-anthrachinon, 2-(2'-Aminoethyl)-amino-anthrachinon, 2-Brom-4,8-diamino-6-(3'-trimethylammonium)-phenylamino-1,5-naphthochinon, 1-(2'-Sulfo-4'-methyl-phenyl)-amino-4-hydroxy-anthrachinon, 1,4-Diamino-anthrachinon, 1-Amino-2-sulfo-4-cyclohexylamino-anthrachinon, 1-Methylamino-4-aminopropylamino-anthrachinon, 1-Aminopropylamino-anthrachinon, 1,4-Diamino-2-methoxy-anthrachinon und 1,4-Bis(2-Hydroxyethyl)amino-5,8-dihydroxy-anthrachinon.

Beispiele für geeignete Triphenylmethanfarbstoffe sind 4',4'',4'''-Triamino-3-methyl-triphenylcarboniumchlorid, Bis-(4,4-Diethylaminophenyl)-4'-ethylamino-naphthyl-carboniumchlorid, Bis-(4,4-Dimethylaminophen)-4'-phenylamino-naphthyl-carboniumchlorid (Basic Blue 26, CI 44045) und 4,4-Bis-(N-Ethyl-3-sulfobenzyl)-amino-2''-sulfofuchsonium.

Die genannten direktziehenden Nitrofarbstoffe, Azofarbstoffe, Anthrachinonfarbstoffe oder Triphenylmethanfarbstoffe können in einer Gesamtmenge von 0,01 bis 4 Gewichtsprozent enthalten sein.

Die Verbindungen nach Formel (I) können grundsätzlich auch zum Färben anderer Naturfasern, wie zum Beispiel Baumwolle, Jute, Sisal, Leinen oder Seide, modifizierte Naturfasern, wie zum Beispiel Regeneratcellulose, Nitrocellulose, Alkylcellulose oder Hydroxyalkylcellulose oder Acetylcellulose und synthetische Fasern, wie zum Beispiel Polyamidfasern, Polyurethanfasern oder Polyesterfasern dienen.

Die Zubereitungsform des erfindungsgemäßen Haarfärbemittels kann beispielsweise eine Lösung, insbesondere eine wäßrige oder wäßrigalkoholische Lösung, sein. Die besonders bevorzugten Zubereitungsformen sind jedoch eine Creme, ein Gel, eine tensidhaltige schäumende Lösung (Shampoo, Aerosol), eine Emulsion oder andere wasserhaltige Träger, die für die Anwendung auf dem Haar geeignet sind. Ihre Zusammensetzung stellt eine Mischung der Farbstoffkomponente mit den für solche Zubereitungen üblichen Zusätzen dar. Die Konzentration der Verbindungen nach Formel (I) liegt in den erfindungsgemäßen Haarfärbemitteln zwischen 0,01 und 10 Gewichtsprozent, vorzugsweise zwischen 0,5 und 10 Gewichtsprozent.

Übliche Zusätze in Lösungen, Crèmes, Emulsionen oder Gelen sind zum Beispiel Lösungsmittel wie Wasser, niedere aliphatische einwertige oder mehrwertige Alkohole, deren Ester und Ether, beispielsweise Alkanole, insbesondere mit 1 bis 4 C-Atomen, beispielsweise Ethanol, Propanol oder Isopropanol, Butanol, Isobutanol, zweiwertige und dreiwertige Alkohole, insbesondere solche mit 2 bis 6 C-Atomen, beispielsweise Ethylenglycol, Propylenglycol, 1,3-Propandiol, 1,4-Butandiol, 1,5-Pentandiol, 1,6-Hexandiol, 1,2,6-Hexantriol, Glycerin, Diethylenglycol, Dipropylenglycol, Polyalkylenglycole, wie Triethylenglycol, Polyethylenglycol, Tripropylenglycol, Polypropylenglycol, niedere Alkylether von mehrwertigen Alkoholen, wie Ethylenglycolmonomethylether oder Ethylenglycolmonoethylether oder Ethylenglycolmonopropylether oder Ethylenglycolmonobutylether, Diethylenglycolmonomethylether oder Diethylenglycolmonoethylether, Triethylenglycolmonomethylether oder Triethylenglycolmonoethylether, Ketone und Ketoalkohole, insbesondere solche mit 3 bis 7 C-Atomen, wie zum Beispiel Aceton, Methylethylketon, Diethylketon, Methylisobutylketon, Methylphenylketon, Cyclopentanon, Cyclohexanon und Diacetonalkohol, Ether, wie z.B. Dibutylether, Tetrahydrofuran, Dioxan, Diisopropylether, Ester wie zum Beispiel Ethylformiat, Methylformiat, Methylacetat, Ethylacetat, Propylenacetat, Butylacetat, Phenylacetat, Ethylenglycolmonoethyletheracetat und Essigsäurehydroxyethylester, Amide wie zum Beispiel Dimethylformamid und Dimethylacetamid, N-Methylpyrrolidon, sowie Harnstoff, Tetramethylharnstoff und Thiodiglycol.

Weiterhin können in dem erfindungsgemäßen Haartärbemittel Netzmittel oder Emulgatoren aus den Klassen der anionischen, kationischen, amphoteren, nichtionogenen oder zwitterionischen oberflächenaktiven Substanzen wie Fettalkoholsulfate, Alkansulfonate, Alkylbenzolsulfonate, Alkyltrimethylammoniumsalze, Alkylbetaine, α-Olefinsulfonate, oxethylierte Fettalkohole, oxethylierte Nonylphenole, Fettsäurealkanolamine, oxethylierte Fettsäureester, Fettalkoholpolyglycolethersulfate, Alkylpolyglucoside, Verdickungsmittel wie höhere Fettalkohole, Stärke, Cellulosederivate, Vaseline, Paraffinöl, Fettsäuren und andere Fettkomponenten in emulgierter Form, wasserlösliche polymere Verdickungsmittel wie natürliche Gummen, Guargummi, Xanthangummi, Johannisbrotkernmehl, Pektin, Dextran, Agar-Agar, Amylose, Amylopektin, Dextrine, Tone oder vollsynthetische Hydrokolloide wie Polyvinylalkohol, außerdem Pflegestoffe wie Lanolinderivate, Cholesterin, Pantothensäure, wasserlösliche kationische Polymere, Proteinderivate, Provitamine, Vitamine, Pflanzenextrakte, Zucker und Betain, Hilfsstoffe wie Feuchthaltemittel, Elektrolyte, Antioxidantien, Fettamide, Sequestrierungsmittel, filmbildende Agenten und Konservierungsmittel, enthalten sein. Neben Wasser kann auch auch ein wasserlösliches organisches Lösungsmittel oder ein Gemisch derartiger Lösungsmittel sowie ein Wasser/Lösungsmittel-Gemisch verwendet werden.

Die erwähnten Bestandteile werden in den für solche Zwecke üblichen Mengen verwendet, zum Beispiel die Netzmittel und Emulgatoren in Konzentrationen von etwa 0,5 bis 30 Gewichtsprozent, die Verdicker in einer Menge von etwa 0,1 bis 25 Gewichtsprozent und die Pflegestoffe in einer Menge von etwa 0,1 bis 5 Gewichtsprozent.

Das erfindungsgemäße Mittel weist in gebrauchsfertigen Zustand einen pH-Wert von etwa 5,0 bis 8,0 auf. Der bevorzugte pH-Wert zur Haarfärbung ist der saure Bereich (pH<7), wobei -falls erforderlich- die Einstellung dieses pH-Wertes vorzugsweise mit schwachen organischen Säuren, wie zum Beispiel Zitronensäure, Glykolsäure, Milchsäure, Äpfelsäure, Ascorbinsäure oder Weinsäure erfolgt.

Man läßt die Färbelösung bei 20 bis 50 °C etwa 10 bis 45 Minuten lang, vorzugsweise bei 40 °C 40 Minuten lang, auf das Haar einwirken, spült sodann das Haar mit Wasser aus und trocknet es. Gegebenenfalls wird im Anschluß an diese Spülung mit einem Shampoo gewaschen und/oder mit einer schwachen organische Säure nachgespült. Anschließend wird das Haar getrocknet.

Das erfindungsgemäße Haarfärbemittel führt zu Haarfärbungen mit ausgezeichneten Echtheitseigenschaften, insbesondere was die Lichtechtheit, Waschechtheit und Reibechtheit anbetrifft. Bemerkenswert ist die große Farbintensität und die Farbreinheit der erzielbaren Färbungen. Schließlich ist mit Hilfe des erfindungsgemäßen Haarfärbemittels auch eine Anfärbung von ergrautem und chemisch nicht vorgeschädigtem Haar problemlos und mit sehr guter Deckkraft möglich. Die dabei erhaltenen Färbungen sind, unabhängig von der unterschiedlichen Struktur des Haares, gleichmäßig und sehr gut reproduzierbar.

Die nachfolgenden Beispiele sollen den Erfindungsgegenstand näher erläutern, ohne diesen jedoch darauf zu beschränken.

### Beispiele

### I. Herstellungsbeispiele

Die Verbindungen nach Formel (I) werden durch Umsetzung der entsprechenden Nitrohalogenbenzole mit einer geeigneten CH-aziden Verbindung dargestellt. Die experimentellen Vorschriften dieser Herstellungsmethode werden unter anderem von C.A. Grob und O. Weissbach in Helvetica Chimica Acta 44, Seite 1748 (1961) oder von H. R. Snyder, E. P. Merica, C. G. Force and E. G. White im Journal of the American Chemical Society 4622, Seite 80 (1958) beschrieben.

### Beispiel 1: Cyano-(2,4-dinitro-phenyl)-essigsäureethylester

11,8 mmol Natriumethylat werden in Ethanol gelöst. Unter Kühlung werden bei 0° C nacheinander Diethylether und 10,74 mmol Ethylcyanoacetat zugegeben. Anschliessend wird langsam eine Lösung von 5,37 mmol 2,4-Dinitro-1-fluor-benzol in Diethylether zugegeben. Das Reaktionsgemisch wird mit Eiswasser und Diethylether aufgenommen und mit 2n HCl- und gesättigter NaCl-Lösung gewaschen. Die vereinigten organischen Phasen werden über Magnesiumsulfat getrocknet, filtriert und im Rotationsverdampfer unter vermindertem Druck (Wasserstrahlvakuum) eingeengt. Man erhält ein brillantrotes Öl (80% der Theorie), das im Hochvakuum (p = 0,05 mm Hg) fest wird und durch präparative Dünnschichtchromatographie gereinigt werden kann. Das Produkt schmilzt bei 58 bis 64° C und besitzt eine Reinheit (HPLC) von 99,7%.
DC(Hexan/ Ethylacetat 3:1): *R*_{f}=0,38.
UV-VIS (nm; c = 13,3 mg/ l; Lösungsmittel: 60% Phospatpuffer (A) (bestehend aus 90 Teilen einer Lösung von 13,6 g Kaliumdihydrogenphosphat und 0,61 g Natriumheptansulfonat in 2 l Acetonitril und 10 Teilen Acetonitril, pH-Wert mit Triethylamin auf 6,1 eingestellt) und 40% Acetonitril(B): λₘₐₓ = 475.
¹H-NMR (500 MHz, DMSO-d₆): δ= 1,13 (t, CH₃, 3H); 3,34 (s, CH, 1H); 3,93 (q, CH₂, 2H); 7,55 (d, H-(C6), 1H); 7,95 (dxd, H-C(5), 1H); 8,27 (d, H-C(3), 1H).
¹³C-NMR (500 MHz, DMSO-d₆): 14,79 (CH₃); 58,12 (CH₂); 63,71 (CH); 122,74 (CN); 122,13, 124,91, 125,27 (C3, C5, C6); 135,84, 140,45, 142,91 (C1, C2, C4); 166,23 (C=O).
IR (Film): 3110w, 2987m, 2250w, 1749vs, 1610s, 1542s, 1468s, 1350s, 1261s, 1216s, 1026s.
MS (m/z, rel. Intensität): 279 (3, M⁺), 233 (97, -NO₂), 206 (100, -COOEt), 189 (55), 179 (60), 159 (15), 134 (58), 114 (49), 29 (72).

| CHN-Analalyse: (C₁₁H₉O₆N₃) | | | |
|---|---|---|---|
| berechnet: | C 47,32 | H 3,25 | N 15,05 |
| gefunden: | C 48,38 | H 3,91 | N 14,45 |

### Beispiel 2: 2-(2,4-Dinitro-phenyl)-malonsäuredinitril

11,8 mmol Natriumethylat werden in Ethanol gelöst. Unter Kühlung werden bei 0° C nacheinander Diethylether und 10,74 mmol Malonsäuredinitril zugegeben. Anschliessend wird langsam eine Lösung von 5,37 mmol 2,4-Dinitro-1-fluor-benzol in Diethylether zugegeben. Das Reaktionsgemisch wird mit Eiswasser und Diethylether aufgenommen und mit 2n HCl- und gesättigter NaCl-Lösung gewaschen. Die vereinigten organischen Phasen werden über Magnesiumsulfat getrocknet, filtriert und im Rotationsverdampfer unter vermindertem Druck (Wasserstrahlvakuum) eingeengt. Man erhält ein blassoranges Öl (60% der Theorie).
DC (Hexan/ Ethylacetat 3:1): *R*_{f}=0,31.
¹H-NMR (500 MHz, DMSO-d₆): 3,34 (s, CH, 1H); 7,55 (d, H-(C6), 1H); 7,95 (dxd, H-C(5), 1H); 8,27 (d, H-C(3), 1H).

### Beispiel 3: 2-(2,4-Dinitro-phenyl)-3-oxo-butyryl-essigsäureethylester

11,8 mmol Natriumethylat werden in Ethanol gelöst. Unter Kühlung werden bei 0° C nacheinander Diethylether und 10,74 mmol Acetessigsäureethylester hinzugefügt. Anschliessend wird langsam eine Lösung von 5,37 mmol 2,4-Dinitro-1-fluor-benzol in Diethylether zugegeben. Das Reaktionsgemisch wird mit Eiswasser und Diethylether aufgenommen und mit 2n HCl- und gesättigter NaCl-Lösung gewaschen. Die vereinigten organischen Phasen werden über Magnesiumsulfat getrocknet, filtriert und im Rotationsverdampfer unter vermindertem Druck (Wasserstrahlvakuum) eingeengt. Man erhält einen gelben Feststoff (55% der Theorie).
Schmelzpunkt.: 62-66° C.
DC (Hexan/ Ethylacetat 3:1): *R*_{f} =0,30.

### Beispiel 4: (5-Amino-2,4-dinitro-phenyl)-cyanoessigsäureethylester

11,8 mmol Natriumethylat werden in Ethanol gelöst. Unter Kühlung werden bei 0° C nacheinander Diethylether und 10,74 mmol Ethylcyanoacetat zugegeben. Anschliessend wird langsam eine Lösung von 5,37 mmol 2,4-Dinitro-5-fluor-anilin in Diethylether zugegeben. Das Reaktionsgemisch wird mit Eiswasser und Diethylether aufgenommen und mit 2n HCl- und gesättigter NaCl-Lösung gewaschen. Die vereinigten organischen Phasen werden über Magnesiumsulfat getrocknet, filtriert und im Rotationsverdampfer unter vermindertem Druck (Wasserstrahlvakuum) eingeengt. Man erhält einen braunen Feststoff (76% der Theorie).
Schmelzpunkt.: 66-68° C.
DC (Hexan/ Ethylacetat 3:1): *R*_{f}=0,38.

### Beispiel 5: Cyano-[2,4-Dinitro-5-(cyano-ethoxycarbonylmethyl)-phenyl]-essigsäureethylester

23,6 mmol Natriumethylat werden in Ethanol gelöst. Unter Kühlung werden bei 0° C nacheinander Diethylether und 21,5 mmol Ethylcyanoacetat zugegeben. Anschliessend wird langsam eine Lösung von 5,37 mmol 2,4-Dinitro-1,5-difluor-benzol in Diethylether zugegeben. Das Reaktionsgemisch wird mit Eiswasser und Diethylether aufgenommen und mit 2n HCl- und gesättigter NaCl-Lösung gewaschen. Die vereinigten organischen Phasen werden über Magnesiumsulfat getrocknet, filtriert und im Rotationsverdampfer unter vermindertem Druck (Wasserstrahlvakuum) eingeengt. Man erhält ein orange-braunes Öl (80% der Theorie).
DC (Hexan/ Ethylacetat 3:1): *R*_{f}=0,35.
¹H-NMR (500 MHz, DMSO-d₆): 1,15 (2xt, CH₃, 2x3H); 3,34 (s, CH, 2x1H); 3,98 (q, CH₂, 2H); 4,18 (q, CH₂, 2H); 7,70 (d, H-(C6), 1H); 8,41 (d, H-C(3), 1H).

### Beispiel 6: 5-(2,4-Dinitrophenyl)-pyrimidin-2,4,6-trion

11,8 mmol Natriumethylat werden in Ethanol gelöst. Unter Kühlung werden bei 0° C nacheinander Diethylether und 10,74 mmol 2,4,6-Trihydroxypyrimidin zugegeben. Anschliessend wird langsam eine Lösung von 5,37 mmol 2,4-Dinitro-1-fluor-benzol in Diethylether zugegeben. Das Reaktionsgemisch wird mit Eiswasser und Diethylether aufgenommen und mit 2n HCl- und gesättigter NaCl-Lösung gewaschen. Die vereinigten organischen Phasen werden über Magnesiumsulfat getrocknet, filtriert und im Rotationsverdampfer unter vermindertem Druck (Wasserstrahlvakuum) eingeengt. Man erhält einen hellbraunen Feststoff (20% der Theorie).
DC (Hexan/ Ethylacetat 3:1): *R*_{f}=0,30.

### Beispiel 7: Cyano-4-nitrophenyl-essigsäureethylester

11,8 mmol Natriumethylat werden in Ethanol gelöst. Unter Kühlung werden bei 0° C nacheinander Diethylether und 10,74 mmol Ethylcyanoacetat zugegeben. Anschliessend wird langsam eine Lösung von 5,37 mmol 4-Nitro-chlorbenzol in Diethylether zugegeben. Das Reaktionsgemisch wird mit Eiswasser und Diethylether aufgenommen und mit 2n HCl- und gesättigter NaCl-Lösung gewaschen. Die vereinigten organischen Phasen werden über Magnesiumsulfat getrocknet, filtriert und im Rotationsverdampfer unter vermindertem Druck (Wasserstrahlvakuum) eingeengt. Man erhält nach Chromatographie einen gelben Feststoff (50% der Theorie).
¹H-NMR (60 MHz, DMSO-d₆): 1,25 (t, CH₃, 3H); 3,34 (s, CH, 1H); 4,25 (q, CH₂, 2H); 7,60-7,80, 8,15-8,35 (2 dxd, H-(C6), H-C(5), H-C(2), H-C(3), 4H).

### Beispiel 8: 2-(4-Nitrophenyl)-malonsäurediethylester

11,8 mmol Natriumethylat werden in Ethanol gelöst. Unter Kühlung werden bei 0° C nacheinander Diethylether und 10,74 mmol Malonsäurediethylester zugegeben. Anschliessend wird langsam eine Lösung von 5,37 mmol 4-Nitro-chlorbenzol in Diethylether zugegeben. Das Reaktionsgemisch wird mit Eiswasser und Diethylether aufgenommen und mit 2n HCl- und gesättigter NaCl-Lösung gewaschen. Die vereinigten organischen Phasen werden über Magnesiumsulfat getrocknet, filtriert und im Rotationsverdampfer unter vermindertem Druck (Wasserstrahlvakuum) eingeengt. Man erhält nach Chromatographie einen gelben Feststoff (32% der Theorie).
¹H-NMR (60 MHz, DMSO-d₆): 1,35 (dxt, 2xCH₃, 6H); 3,35 (s, CH, 1H); 4,25 (dxq, 2xCH₂, 4H); 6,80-7,05, 8,15-8,35 (2 dxd, H-(C6), H-C(5), H-C(2), H-C(3), 4H).

### II. Beispiele für Haarfärbemittel

### Beispiel 9: Haarfärbemittel mit Cyano-(2,4-dinitrophenyl)essigsäureethylester

- 0,0698 g: Cyano-(2,4-dinitrophenyl)-essigsäureethylester
- 1,0000 g: Isopropanol
- 0,1000 g: Benzylalkohol
- 1,0000 g: Laurylalkohol-diglycolethersulfat-natriumsalz (28%ige wässerige Lösung)
- ad 10,0000 g: Wasser

Das vorstehende gebrauchsfertige Haarfärbemittel wird unmittelbar vor Gebrauch durch Vermischen des Farbstoffes mit den restlichen Komponenten erhalten. Die Einstellung des pH-Wertes erfolgt entweder mit Milchsäure oder mit verdünnter Ammoniaklösung. Gebleichte Haare werden sodann 40 Minuten bei einer Temperatur von 40° C mit den Haarfärbemittel (einmal mit Milchsäure sauer und einmal mit verdünnter Ammoniaklösung basisch eingestellt) behandelt. Anschließend werden die Haare mit Wasser gespült und getrocknet.

### Beispiel 10: Haarfärbemittel mit 2-(2,4-Dinitrophenyl)-malonodinitril

- 0,0581 g: 2-(2,4-Dinitrophenyl)-malonodinitril
- 1,0000 g: Isopropanol
- 0,1000 g: Benzylalkohol
- 1,0000 g: Laurylalkohol-diglycolethersulfat-natriumsalz (28%ige wässerige Lösung)
- ad 10,0000 g: Wasser

Das vorstehende gebrauchsfertige Haarfärbemittel wird unmittelbar vor Gebrauch durch Vermischen des Farbstoffes mit den restlichen Komponenten erhalten. Die Einstellung des pH-Wertes erfolgt entweder mit Milchsäure oder mit verdünnter Ammoniaklösung. Gebleichte Haare werden sodann 40 Minuten bei einer Temperatur von 40° C mit den Haarfärbemittel (einmal mit Milchsäure sauer und einmal mit verdünnter Ammoniaklösung basisch eingestellt) behandelt. Anschließend werden die Haare mit Wasser gespült und getrocknet.

### Beispiel 11: Haarfärbemittel mit 2-(2,4-Dinitrophenyl)-3-oxo-butyrylessigsäureethylester

- 0,0741 g: 2-(2,4-Dinitrophenyl)-3-oxo-butyrylessigsäureethylester
- 1,0000 g: Isopropanol
- 0,1000 g: Benzylalkohol
- 1,0000 g: Laurylalkohol-diglycolethersulfat-natriumsalz (28% wässerige Lösung)
- ad 10,0000 g: Wasser

Das vorstehende gebrauchsfertige Haarfärbemittel wird unmittelbar vor Gebrauch durch Vermischen des Farbstoffes mit den restlichen Komponenten erhalten. Die Einstellung des pH-Wertes erfolgt entweder mit Milchsäure oder mit verdünnter Ammoniaklösung. Gebleichte Haare werden sodann 40 Minuten bei einer Temperatur von 40° C mit den Haarfärbemittel (einmal mit Milchsäure sauer und einmal mit verdünnter Ammoniaklösung basisch eingestellt) behandelt. Anschließend werden die Haare mit Wasser gespült und getrocknet.

### Beispiel 12: Haarfärbemittel mit (5-Amino-2,4-dinitrophenyl)-cyanoessigsäureethylester

- 0,0736 g: (5-Amino-2,4-dinitrophenyl)-cyanoessigsäureethylester
- 1,0000 g: Isopropanol
- 0,1000 g: Benzylalkohol
- 1,0000 g: Laurylalkohol-diglycolethersulfat-natriumsalz (28%ige wässerige Lösung)
- ad 10,0000 g: Wasser

Das vorstehende gebrauchsfertige Haarfärbemittel wird unmittelbar vor Gebrauch durch Vermischen des Farbstoffes mit den restlichen Komponenten erhalten. Die Einstellung des pH-Wertes erfolgt entweder mit Milchsäure oder mit verdünnter Ammoniaklösung. Gebleichte Haare werden sodann 40 Minuten bei einer Temperatur von 40° C mit den Haarfärbemittel (einmal mit Milchsäure sauer und einmal mit verdünnter Ammoniaklösung basisch eingestellt) behandelt. Anschließend werden die Haare mit Wasser gespült und getrocknet.

### Beispiel 13: Haarfärbemittel mit Cyano-[2,4-Dinitro-5-(cyano-ethoxycarbonylmethyl)-phenyl]-essigsäureethylester

- 0,0976 g: Cyano-[2,4-Dinitro-5-(cyano-ethoxycarbonylmethyl)-phenyl]-essigsäureethylester
- 1,0000 g: Isopropanol
- 0,1000 g: Benzylalkohol
- 1,0000 g: Laurylalkohol-diglycolethersulfat-natriumsalz (28%ige wässerige Lösung)
- ad 10,0000 g: Wasser

Das vorstehende gebrauchsfertige Haarfärbemittel wird unmittelbar vor Gebrauch durch Vermischen des Farbstoffes mit den restlichen Komponenten erhalten. Die Einstellung des pH-Wertes erfolgt entweder mit Milchsäure oder mit verdünnter Ammoniaklösung. Gebleichte Haare werden sodann 40 Minuten bei einer Temperatur von 40° C mit den Haarfärbemittel (einmal mit Milchsäure sauer und einmal mit verdünnter Ammoniaklösung basisch eingestellt) behandelt. Anschließend werden die Haare mit Wasser gespült und getrocknet.

### Beispiel 14: Haarfärbemittel mit 5-(2,4-Dinitrophenyl)-pyrimidin-2,4,6-trion

- 0,0733 g: 5-(2,4-Dinitrophenyl)-pyrimidin-2,4,6-trion
- 1,0000 g: Isopropanol
- 0,1000 g: Benzylalkohol
- 1,0000 g: Laurylalkohol-diglycolethersulfat-natriumsalz (28%ige wässerige Lösung)
- ad 10,000 g: Wasser

Das vorstehende gebrauchsfertige Haarfärbemittel wird unmittelbar vor Gebrauch durch Vermischen des Farbstoffes mit den restlichen Komponenten erhalten. Die Einstellung des pH-Wertes erfolgt entweder mit Milchsäure oder mit verdünnter Ammoniaklösung. Gebleichte Haare werden sodann 40 Minuten bei einer Temperatur von 40° C mit den Haarfärbemittel (einmal mit Milchsäure sauer und einmal mit verdünnter Ammoniaklösung basisch eingestellt) behandelt. Anschließend werden die Haare mit Wasser gespült und getrocknet.

### Beispiel 15: Haarfärbemittel mit Cyano-4-nitrophenyl-essigsäureethylester

- 0,0698 g: Cyano-4-nitrophenyl-essigsäureethylester
- 1,0000 g: Isopropanol
- 0,1000 g: Benzylalkohol
- 1,0000 g: Laurylalkohol-diglycolethersulfat-natriumsalz (28%ige wässerige Lösung)
- ad 10,0000 g: Wasser

Das vorstehende gebrauchsfertige Haarfärbemittel wird unmittelbar vor Gebrauch durch Vermischen des Farbstoffes mit den restlichen Komponenten erhalten. Die Einstellung des pH-Wertes erfolgt entweder mit Milchsäure oder mit verdünnter Ammoniaklösung. Gebleichte Haare werden sodann 40 Minuten bei einer Temperatur von 40° C mit den Haarfärbemittel (einmal mit Milchsäure sauer und einmal mit verdünnter Ammoniaklösung basisch eingestellt) behandelt. Anschließend werden die Haare mit Wasser gespült und getrocknet.

### Beispiel 16: Haarfärbemittel mit 2-(4-Nitrophenyl)-malonsäurediethylester

- 0,0698 g: 2-(4-Nitrophenyl)-malonsäurediethylester
- 1,0000 g: Isopropanol
- 0,1000 g: Benzylalkohol
- 1,0000 g: Laurylalkohol-diglycolethersulfat-natriumsalz (28%ige wässerige Lösung)
- ad 10,0000 g: Wasser

Das vorstehende gebrauchsfertige Haarfärbemittel wird unmittelbar vor Gebrauch durch Vermischen des Farbstoffes mit den restlichen Komponenten erhalten. Die Einstellung des pH-Wertes erfolgt entweder mit Milchsäure oder mit verdünnter Ammoniaklösung. Gebleichte Haare werden sodann 40 Minuten bei einer Temperatur von 40° C mit den Haarfärbemittel (einmal mit Milchsäure sauer und einmal mit verdünnter Ammoniaklösung basisch eingestellt) behandelt. Anschließend werden die Haare mit Wasser gespült und getrocknet.

Die erhaltenen Färbeergebnisse sind in Tabelle 1 zusammengefaßt.

**Tabelle 1**

| Übersicht der mit Nitrobenzylderivaten der Formel (I) ohne Zusatzfarbstoffe erhaltenen Ausfärbungen: | | |
|---|---|---|
| | **Färbeergebnis** | |
| **Beispiel** | **pH 5 (Milchsäure)** | **pH 8 (verd. Ammoniak)** |
| **9** | kräftig orangerot | orange |
| **10** | hellbraun-orange | hellbraun |
| **11** | hellorange | hellorange |
| **12** | orangerot | orange |
| **13** | intensiv orangerot | orange |
| **14** | hellbraun | hellbraun |
| **15** | intensiv rot | rot |
| **16** | intensiv rot | rot |

Die erhaltenen Färbungen überstehen bis zu fünf Haarwäschen oder eine dreiwöchige Exposition unter Sonnenlicht ohne merklichen Intensitätsverlust.

### Beispiele 17 - 40: Haarfärbemittel mit Cyano-(2,4-dinitrophenyl)-essigsäureethylester und Zusatzfarbstoffen

- 0,0698 g: Cyano-(2,4-dinitrophenyl)-essigsäureethylester
- X g: Zusatzfarbstoffe gemäß **Tabelle 2**
- 1,0000 g: Isopropanol
- 0,1000 g: Benzylalkohol
- 1,0000 g: Laurylalkohol-diglycolethersulfat-natriumsalz (28%ige wässerige Lösung)
- ad 10,0000 g: Wasser

Das vorstehende gebrauchsfertige Haarfärbemittel wird unmittelbar vor Gebrauch durch Vermischen der Farbstoffe mit den restlichen Komponenten erhalten. Die Menge der Zusatzfarbstoffe beträgt je nach Art des verwendeten Zusatzfarbstoffes je nach Beispiel 0,05 bis 0,6 Gewichtsprozent. In den Beispielen 17-28 beträgt das mengenmäßige Verhältnis Cyano-(2,4-dinitrophenyl)essigsäureethylester zu Zusatzfarbstoff 2:1. In den Beispielen 29-40 beträgt das mengenmäßige Verhältnis A:B:C, wobei A für den Cyano-(2,4-dinitrophenyl)-essigsäureethylester und B und C für die Zusatzfarbstoffe stehen. Die Einstellung der pH-Werte wird einmal mit Milchsäure und einmal mit verdünnter Ammoniaklösung vorgenommen. Gebleichte Haare werden sodann 40 Minuten bei einer Temperatur von 40° C mit den Haarfärbe-mitteln (je einmal sauer und basisch eingestellt) behandelt. Anschließend werden die Haare mit Wasser gespült und getrocknet.

Die erhaltenen Färbeergebnisse sind in Tabelle 2 zusammengefaßt.

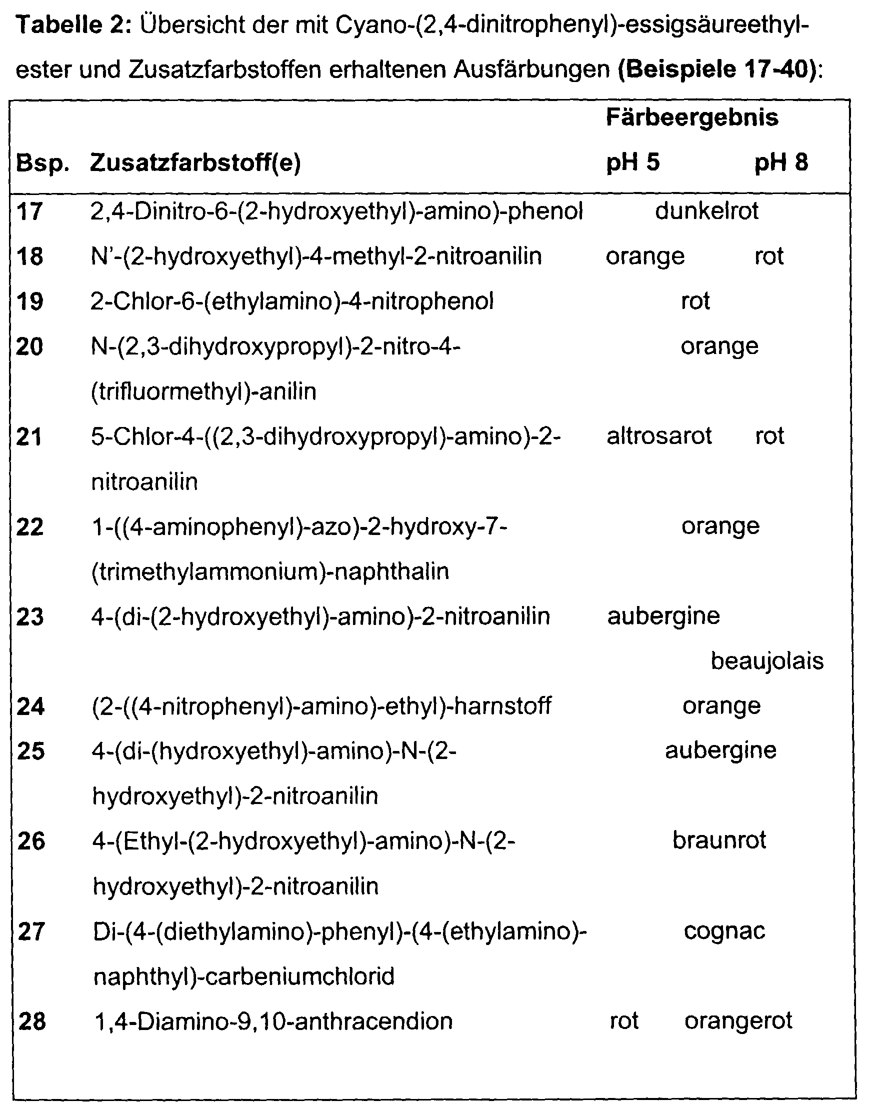

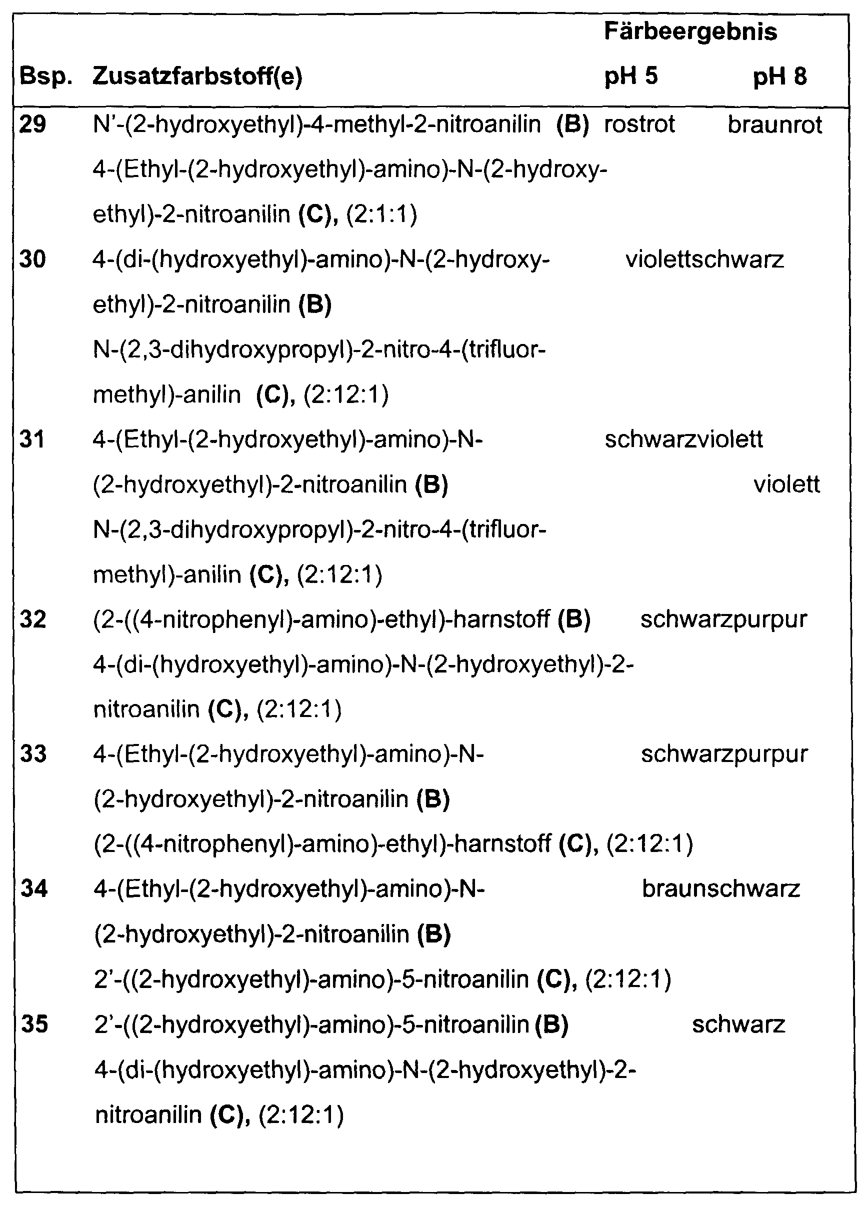

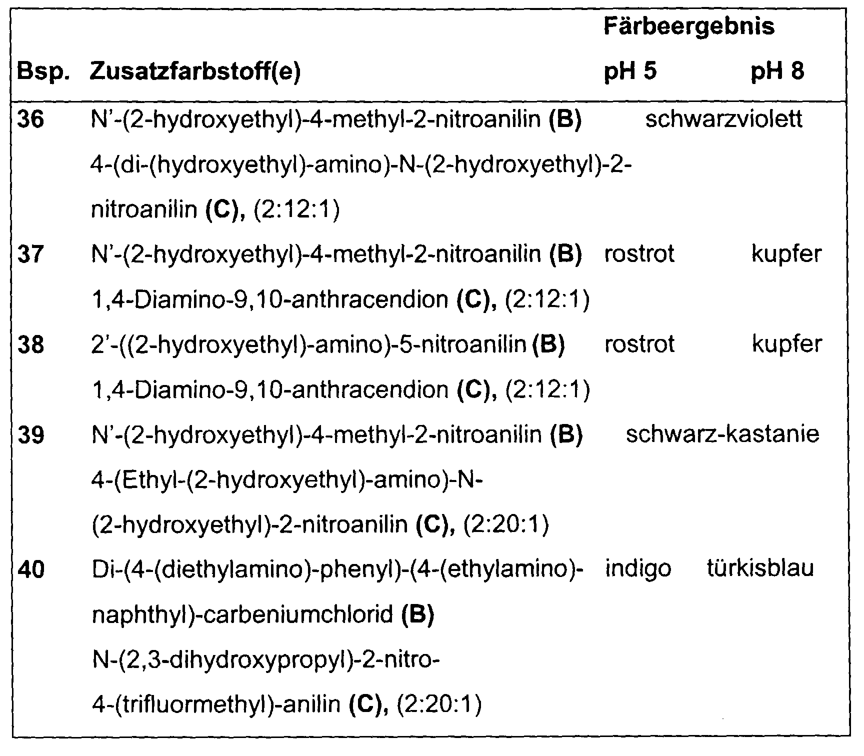

Alle Prozentangeben stellen, soweit nicht anders angegeben, Gewichtsprozente dar.

## Patentansprüche

1. Mittel zur Färbung keratinischer Fasern, dadurch gekennzeichnet, daß es mindestens ein Nitrobenzylderivat der Formel (I), 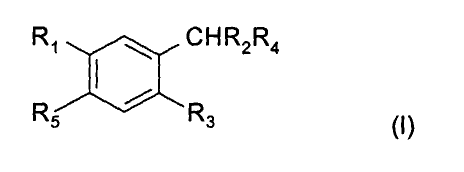 worin unabhängig voneinander gilt:
R₁ = H, NH₂, NHRₓ, NRₓ, NO₂, CH(CN)₂, CONHRₓ,
OH, ORₓ, OCORₓ, CHRₓCOORₓ, CHCNCOORₓ oder CORₓ
mit Rₓ = H, Phenyl oder einer niederen Alkylgruppe mit
1-4 C Atomen;
R₂ = CN, CONHRₓ, NHCORₓ, CORₓ, COORₓ, Trihydroxypyrimidinyl oder 4,6-Dihydroxy-2-mercaptopyrimidinyl
mit Rₓ = H, Phenyl oder einer niederen Alkylgruppe mit
1-4 C Atomen;
R₄ = CN, CONHRₓ, NHCORₓ, CORₓ oder COORₓ
mit Rₓ = H, Phenyl oder einer niederen Alkylgruppe mit
1-4 C Atomen; und
R₃ = H oder NO₂,
R₅ = H oder NO₂ , wobei R₃ und R₅ nicht gleichzeitig H bedeuten; enthält.

2. Mittel nach Anspruch 1 dadurch gekennzeichnet, daß die Verbindung der Formel (I) ausgewählt ist aus Cyano-(2,4-dinitrophenyl)-essigsäureethylester, (5-Amino-2,4-dinitrophenyl)-cyano-essigsäureethylester, Cyano-[2,4-Dinitro-5-(cyano-ethoxycarbonylmethyl)-phenyl]-essigsäureethylester, 2-(2,4-Dinitrophenyl)-3-oxo-butyrylessigsaureethylester, 2-(2,4-Dinitrophenyl)-malonsäurediethylester, Cyano-4-nitrophenyl-essigsäureethylester, 2-(2,4-Dinitrophenyl)-malonodinitril und 2-(4-Nitrophenyl)-malonsäurediethylester.

3. Mittel nach Anspruch 1 oder 2, dadurch gekennzeichet, daß die Verbindung der Formel (I) in einer Menge von 0,01 bis 10 Gewichtsprozent enthalten ist.

4. Mittel nach einem der Ansprüche 1 bis 3, dadurch gekennzeichnet, daß es zusätzlich mindestens einen direktziehenden Farbstoff aus der Gruppe bestehend aus Nitrofarbstoffen, Azofarbstoffen, Anthrachinonfarbstoffen oder Triphenylmethanfarbstoffen enthält.

5. Mittel nach Anspruch 4, dadurch gekennzeichnet, daß die direktziehenden Farbstoffe in einer Menge von 0,01 bis 4 Gewichtsprozent enthalten sind.

6. Mittel nach einem der Ansprüche 1 bis 5 dadurch gekennzeichnet, daß es für Haarfärbemittel übliche, kosmetisch verträgliche Zusätze enthält.

7. Mittel nach einem der Ansprüche 1 bis 6, dadurch gekennzeichnet, daß es einen pH-Wert von 5,0 bis 8,0 aufweist.

8. Mittel nach Anspruch 7, dadurch gekennzeichnet, daß es einen pH-Wert von kleiner 7 aufweist.

9. Verfahren zur Färbung von Haaren, dadurch gekennzeichnet, daß man eine für die Behandlung ausreichende Menge, je nach Haarfülle im allgemeinen etwa 10 bis 60 g, eines Mittels nach einem der Ansprüche 1 bis 8 auf die Haare aufträgt, es dort 10 bis 45 Minuten lang bei einer Temperatur von 15 bis 50 Grad Celsius einwirken läßt, anschließend die Haare mit Wasser spült und sodann trocknet.

10. Verfahren nach Anspruch 9, dadurch gekennzeichnet, daß das Haar vor dem Trocknen mit einem Shampoo gewaschen und/oder mit einer schwachen organischen Säure gespült wird.
